# EUROPÄISCHE PATENTANMELDUNG

(11) **EP 3 771 712 A1**
(43) Veröffentlichungstag der Anmeldung: **03.02.2021**
(21) Anmeldenummer: 19188967.4
(22) Anmeldetag: 30.07.2019
(51) Int. Cl.: C07D 401/04

(54) **VERFAHREN ZUR HERSTELLUNG STICKSTOFFHALTIGER HETEROCYCLEN**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung betrifft ein verbessertes Verfahren zur Herstellung stickstoffhaltiger Heterocyclen der Formel (I)

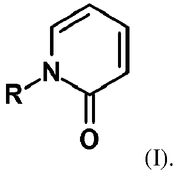

## Beschreibung

Die vorliegende Erfindung betrifft ein verbessertes Verfahren zur Herstellung von stickstoffhaltigen, heterocyclischen Verbindungen der weiter unten gezeigten Formel (I).

Verbindungen der Formel (I) und ihre Verwendung zur Herstellung von Verbindungen mit pestiziden Eigenschaften sind bereits bekannt geworden (siehe WO 2017/005673 A1 und WO 2019/105871 A1).

Bisher wurden diese Verbindungen durch Kupplungsreaktion von Halogen-haltigen Heterocyclen mit 2(1*H*)-Pyridinon in nicht zufriedenstellender Ausbeute erhalten (siehe zum Beispiel die Synthese von [6'-Trifluormethyl-[1(2*H*), 3'-bipyridin]-2-on, WO 2017/005673 A1 oder oder 6'-Difluormethyl-[1(2H), 3'-bipvridin]-2-on, WO 2019/105871 A1).

Nachteilig bei dem vorbekannten Verfahren ist die unbefriedigende Gesamtausbeute und gegebenenfalls die Isolierung und Reinigung der Intermediate, weil das mit einem erheblichen zeitlichen Aufwand, Ausbeuteverlust und Kosten verbunden ist.

In WO 2017/005673 A1 ist die Herstellung von Verbindungen der Formel 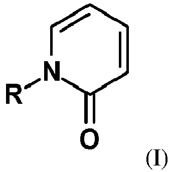 beschrieben, in denen R für einen gegebenenfalls substituierten Heterocyclus steht (Schema 1).

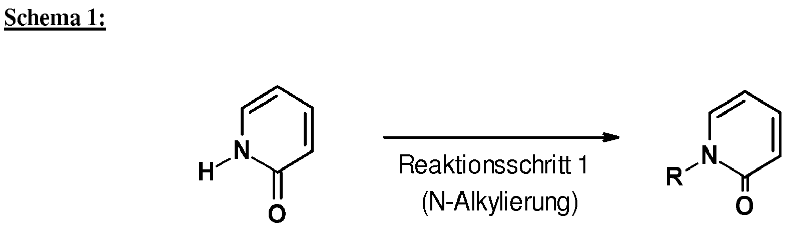

Konkret beschreibt WO 2017/005673 A1 die Herstellung von [6'-Trifluormethyl-[1(2*H*), 3'-bipyridin]-2-on (3), das ausgehend von 2(1*H*)-Pyridinon (1) und 5-Brom-2-trifluormethyl-pyridin (2) in Gegenwart von Kaliumcarbonat und (0,22 mmol) Kupfer(I)-iodid in *N,N-*Dimethylformamid synthetisiert wird (siehe Schema 2).

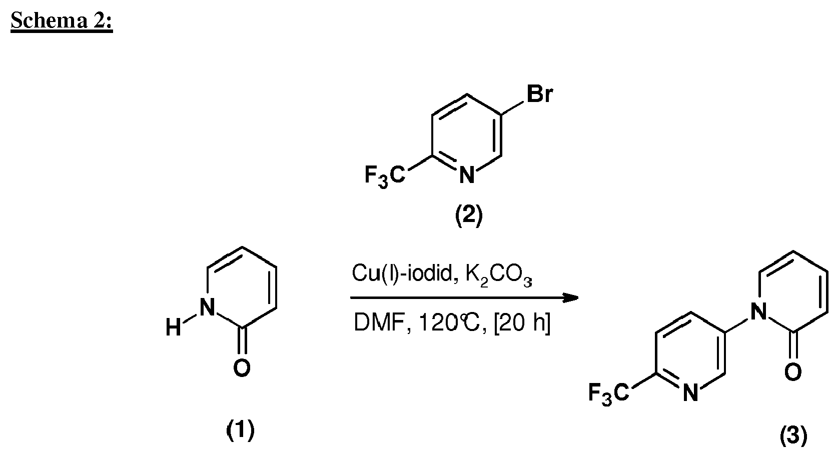

2(1*H*)-Pyridinon (1) wird dabei über einen Zeitraum von 20 Stunden in *N,N*-Dimethylformamid mit der äquimolaren Menge Kaliumcarbonat und einem Überschuss (1,1-fache äquimolare Menge) 5-Brom-2-trifluormethyl-pyridin (2) in Gegenwart von Kupfer(I)-iodid umgesetzt. Die Verbindung (3) wurde in einer Ausbeute von 36 % erhalten.

Dieses Verfahren ist nachteilig, da sich das Halogenatom schlecht gegen die nur schwach basische Amidgruppe (Lactam) des 2(1*H*)-Pyridinons (1) austauschen lässt. Dies führt zu der geringen Ausbeute von lediglich 36 %.

Darüber hinaus erfordert das in WO 2017/005673 A1 beschriebene Verfahren die Verwendung von Halogen-haltigen Ausgangsverbindungen und Metall-haltigen (beispielsweise Kupfer(I)-iodid) Katalysatoren.

Es bestand daher die Notwendigkeit ein Verfahren zur Herstellung der genannten Verbindungen zu finden, das kostengünstig ist und großtechnisch eingesetzt werden kann. Auch ist es erstrebenswert, diese Verbindungen mit hoher Ausbeute und in hoher Reinheit zu erhalten, so dass sie keiner weiteren komplexen Aufreinigung unterzogen werden müssen.

Es wurde nun ein vereinfachtes Verfahren zur Herstellung bestimmter stickstoffhaltiger, heterocyclischer Verbindungen gefunden, das die Nachteile der bekannten Verfahren vermeidet, einfach und kostengünstig durchzuführen ist und großtechnisch eingesetzt werden kann.

Die vorliegende Erfindung betrifft somit ein Verfahren zur Herstellung von Verbindungen der Formel (I) 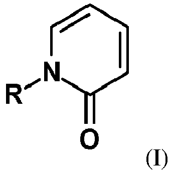 worin
- R: für einen Rest B aus der Reihe 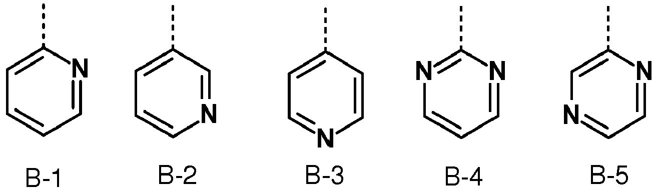 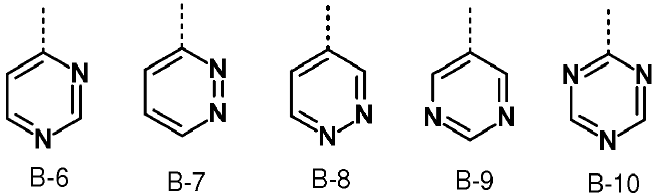 steht, wobei diese Reste n Substituenten Y tragen und die gestrichelte Linie die Bindung zum Stickstoffatom im Rest A bedeutet,
- Y: für einen Rest aus der Reihe Halogen, Cyano, Nitro, Amino, Alkyl, Alkenyl, Alkinyl, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl, Alkoxycarbonyl, Alkylcarbonyl, Cycloalkylcarbonyl, Alkylamino, Dialkylamino, Alkylaminosulfonyl, Dialkylaminosulfonyl, Alkylaminocarbonyl, Dialkylaminocarbonyl, Alkylcarbonylamino, Alkoxyalkylcarbonylamino, Halogenalkylcarbonylamino und jeweils gegebenenfalls substituiertes Aryl und Hetaryl steht und
- n: für eine Zahl aus der Reihe 0, 1 und 2 steht,
das (Methode A) dadurch gekennzeichnet ist, dass in einem ersten Schritt Verbindungen der Formel (II) 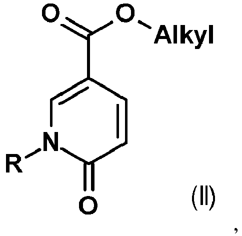 worin Alkyl für C₁-C₄-Alkyl und bevorzugt für Methyl und Ethyl steht und
- R: die oben genannten Bedeutungen hat,
mit einem Alkalimetallhydroxid in Gegenwart eines Verdünnungsmittels in Verbindungen der Formel (III) 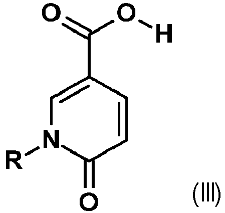 überführt werden und diese anschließend in einem zweiten Reaktionsschritt in Gegenwart eines Metallpulvers, Metalloxids oder Metallcarbonats und einem hoch siedenden Lösungs- oder Verdünnungsmittel zu Verbindungen der Formel (I) umgesetzt werden oder
(Methode B) dadurch gekennzeichnet ist, dass Verbindungen der Formel (II) 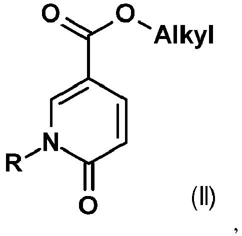 worin
Alkyl für C₁-C₄-Alkyl und bevorzugt für Methyl, Ethyl und tert.-Butyl steht und
- R: die oben genannten Bedeutungen hat,
mit Metallsalzen in einem Lösungs- oder Verdünnungsmittel zu Verbindungen der Formel (I) umgesetzt werden.

Bevorzugt werden Verbindungen der Formel (I) 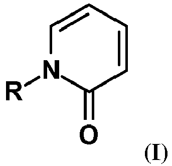 hergestellt, in welchen
- R: für einen Rest B aus der Reihe 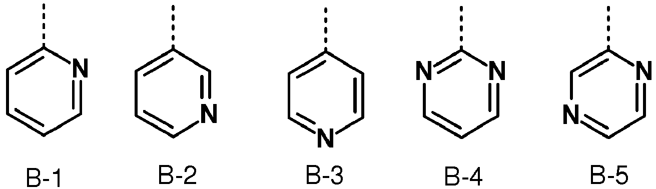 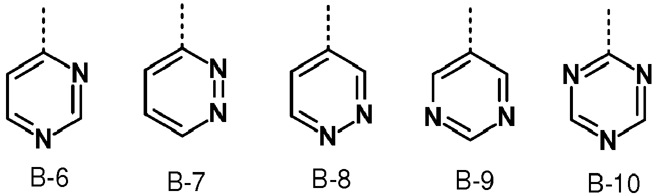 steht, wobei diese Reste n Substituenten Y tragen und die gestrichelte Linie die Bindung zum Stickstoffatom im Rest A bedeutet,
- Y: für einen Rest aus der Reihe Halogen, Cyano, Nitro, Amino, C₁-C₄-Alkyl, C₂-C₄-Alkenyl, C₂-C₄-Alkinyl, Halogen-C₁-C₄-alkyl, C₁-C₄-Alkoxy, Halogen-C₁-C₄-alkoxy, C₁-C₄-Alkylthio, Halogen-C₁-C₄-alkylthio, C₁-C₄-Alkylsulfinyl, Halogen-C₁-C₄-alkylsulfinyl, C₁-C₄-Alkylsulfonyl, Halogen-C₁-C₄-alkylsulfonyl, C₁-C₄-Alkoxycarbonyl, C₁-C₄-Alkylcarbonyl, C₃-C₆-Cycloalkylcarbonyl, C₁-C₄-Alkylamino, Di-(C₁-C₄-alkyl)-amino, C₁-C₄-Alkylaminosulfonyl, Di-(C₁-C₄-alkyl)-aminosulfonyl, C₁-C₄-Alkylaminocarbonyl, D₁-(C₁-C₄-alkyl)-aminocarbonyl, C₁-C₄-Alkylcarbonylamino, C₁-C₄-Alkoxy-C₁-C₄-alkylcarbonylamino, Halogen-C₁-C₄-alkylcarbonylamino, jeweils gegebenenfalls substituiertes Aryl und 5- bis 6-gliedriges Hetaryl steht, und
- n: für eine Zahl aus der Reihe 0, 1 und 2 steht.

Besonders bevorzugt werden Verbindungen der Formel (I) 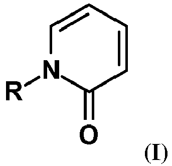 hergestellt, worin (a)
- R: für einen Rest B aus der Reihe 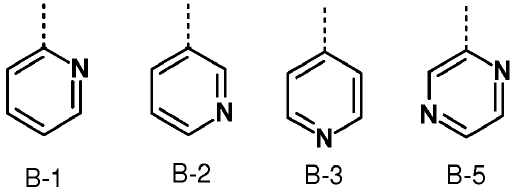 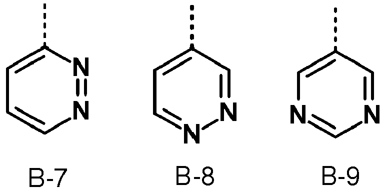 steht, wobei diese Reste n Substituenten Y tragen und die gestrichelte Linie die Bindung zum Stickstoffatom bedeutet,
- Y: für einen Rest aus der Reihe Halogen, Cyano, C₁-C₄-Alkyl, C₂-C₄-Alkenyl, C₂-C₄-Alkinyl, Halogen-C₁-C₄-alkyl, Halogen-C₁-C₄-alkoxy, Halogen-C₁-C₄-alkylthio, C₁-C₄-Alkylsulfinyl, Halogen-C₁-C₄-alkylsulfinyl, C₁-C₄-Alkylsulfonyl, Halogen-C₁-C₄-alkylsulfonyl, gegebenenfalls mit einem Substituenten aus der Reihe Halogen, Cyano, C₁-C₄-Alkyl, Halogen-C₁-C₄-alkyl, Halogen-C₁-C₄-alkoxy, Halogen-C₁-C₄-alkylthio, C₁-C₄-Alkylsulfinyl, Halogen-C₁-C₄-alkylsulfinyl, C₁-C₄-Alkylsulfonyl, Halogen-C₁-C₄-alkylsulfonyl substituiertes 5-gliedriges Hetaryl, wobei Hetaryl beispielsweise *N*-Pyrazolyl, *N*-Imidazolyl oder *N*-1,2,4-Triazolyl bedeutet, steht, und
- n: für eine Zahl aus der Reihe 0, 1 und 2 steht, oder (b)
- R: für den Rest 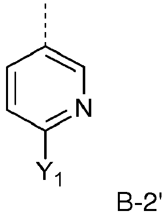
steht,
worin
- Y₁: für einen Rest aus der Reihe Cyano-fluormethyl, Cyano-difluormethyl, Difluormethoxy, Trifluormethoxy, 2,2-Difluorethoxy, 2,2,2-Trifluorethoxy, Difluormethylthio, Trifluormethylthio, Difluormethylsulfinyl, Trifluormethylsulfinyl, Difluormethylsulfonyl, Trifluormethylsulfonyl, H₃C-O-F₂C, H₃C-S-F₂C, H₃C-S(O)-F₂C, H₃C-O₂S-F₂C, H₃C-S-H₂C, H₃C-S(O)-H₂C, H₃C-O₂S-H₂C, 1-Fluorcyclopropyl, 1-Cyanocyclopropyl und 1-Trifluormethylcyclopropyl steht.

Ganz besonders bevorzugt werden Verbindungen der Formel (I) 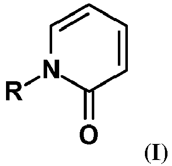 hergestellt, worin (a)
- R: für einen Rest aus der Reihe 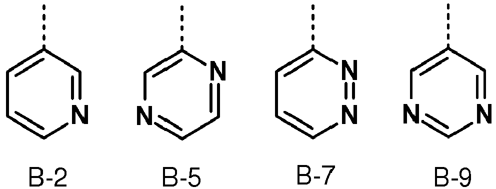 steht, wobei diese Reste n Substituenten Y tragen und die gestrichelte Linie die Bindung zum Stickstoffatom im Rest A bedeutet,
- Y: für einen Rest aus der Reihe Fluor, Chlor, Brom oder Iod, Cyano, Methyl, Fluormethyl, Difluormethyl, Trifluormethyl, Difluorchlormethyl, Difluorbrommethyl, 2,2-Difluorethyl, 2,2,2-Trifluorethyl, Pentafluorethyl, Difluormethoxy, Trifluormethoxy, Difluormethylthio, Trifluormethylthio, Difluormethylsulfinyl, Trifluormethylsulfinyl, Difluormethylsulfonyl, Trifluormethylsulfonyl, N-1,2,4-Triazolyl und *N*-Pyrazolyl, das gegebenenfalls mit einem Substituenten aus der Reihe Fluor, Chlor, Iod, Cyano, Difluormethyl, Trifluormethyl, Difluormethoxy, Trifluormethoxy und Methylthio substituiert ist, steht und
- n: für eine Zahl aus der Reihe 0, 1 und 2 steht, oder (b)
- R: für den Rest 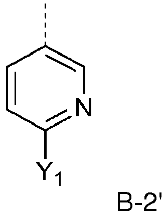
steht,
worin
- Y₁: für einen Rest aus der Reihe Cyano-fluormethyl, Cyano-difluormethyl, Difluormethoxy, Trifluormethoxy, 2,2-Difluorethoxy, 2,2,2-Trifluorethoxy, Difluormethylthio, Trifluormethylthio, Difluormethylsulfinyl, Trifluormethylsulfinyl, Difluormethylsulfonyl, Trifluormethylsulfonyl, H₃C-O-F₂C, H₃C-S-F₂C, H₃C-S(O)-F₂C, H₃C-O₂S-F₂C, H₃C-S-H₂C, H₃C-S(O)-H₂C, H₃C-O₂S-H₂C, 1-Fluorcyclopropyl, 1-Cyanocyclopropyl und 1-Trifluormethylcyclopropyl steht.

Auch ganz besonders bevorzugt werden Verbindungen der Formel (I) 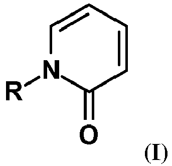 hergestellt, worin
- R: für den Rest 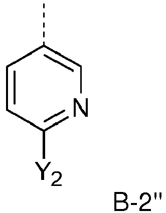
steht und
- Y₂: für einen Rest aus der Reihe, Difluormethyl, Trifluormethyl, Difluorchlormethyl, Difluorbrommethyl, 2,2-Difluorethyl und 2,2,2-Trifluorethyl steht.

Die Verbindungen der Formel (I) lassen sich mit dem erfindungsgemäßen Verfahren mit guten Ausbeuten herstellen.

Die Reaktionsfolge zur Herstellung von Verbindungen der Formel (I) ist im Schema 3 dargestellt. Alkyl steht hier beispielhaft für Methyl, R hat die oben genannte Bedeutung:

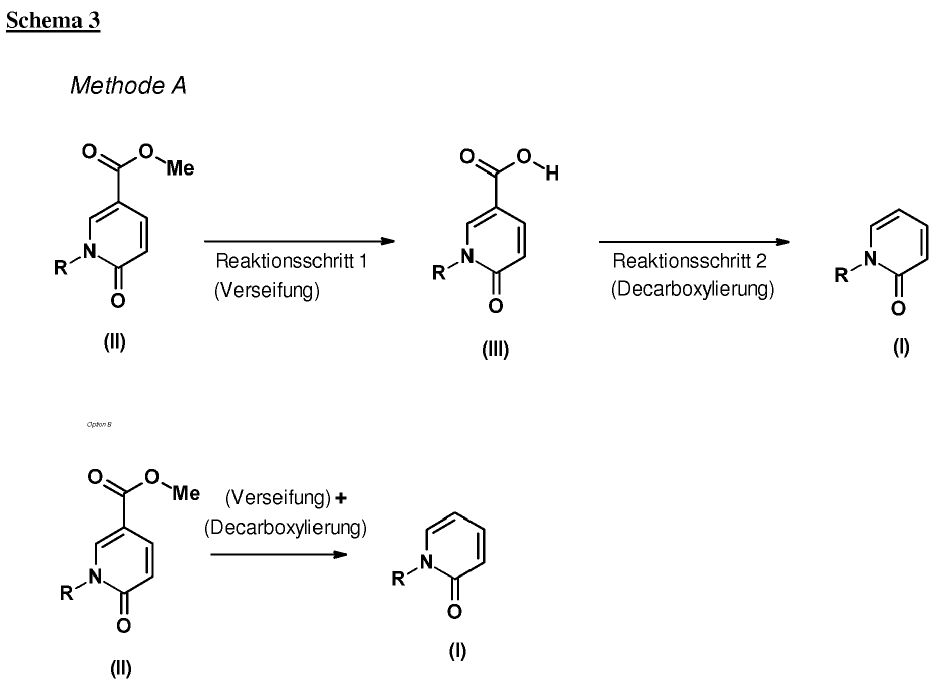

### Methode A.

Nach Methode A reagieren Verbindungen der Formel (II) im ersten Reaktionsschritt in Gegenwart eines geeigneten Alkalimetallhydroxids unter Bildung von Carbonsäuren der Formel (III), die dann im zweiten Reaktionsschritt zu Verbindungen der Formel (I) decarboxylieren.

Einige Verbindungen der Formel (II), in denen R die oben genannten Bedeutungen hat, sind bekannt. Die Herstellung dieser Verbindungen kann nach dem aus WO 2017/005673 A1 bekannten Verfahren erfolgen oder nach A. R. Casola, et al., J. Pharm. Sci. 1965, 54(11), 1686-1688 (B-1); M. S. Akhtar et al. New J. Chem., 2017, 41, 13027-13035 (via Kupfer(II)-katalysierter [3+2+1] Annelierung) (B-1, B-4, B-5, B-7, B-9). Beispielhaft seien folgende Verbindungen der Formel (II) genannt:
- 6-Oxo-[1(6H), 2'-bipyridin]-3-carbonsäuremethylester (R = B-1)
- 6-Oxo-[1(6H), 2'-bipyridin]-3-carbonsäureethylester (B-1)1,6-Dihydro-6-oxo-1-(2-pyrimidinyl)-3-pyridincarbonsäuremethylester (R = B-4)
- 1,6-Dihydro-6-oxo-1-(2-pyrimidinyl)-3-pyridincarbonsäureethylester (B-4)1,6-Dihydro-6-oxo-1-(2-pyridazinyl)-3-pyridincarbonsäuremethylester (R = B-5)
- 1,6-Dihydro-6-oxo-1-(2-pyridazinyl)-3-pyridincarbonsäureethylester (R = B-5)
- 1,6-Dihydro-6-oxo-1-(3-pyridazinyl)-3-pyridincarbonsäuremethylester (R = B-7)
- 1,6-Dihydro-6-oxo-1-(3-pyridazinyl)-3-pyridincarbonsäureethylester (R = B-7)
- 1,6-Dihydro-6-oxo-1-(5-pyrimidinyl)-3-pyridincarbonsäuremethylester (R = B-9)

Bei dem Verfahren nach Methode A wird in einem ersten Reaktionsschritt kommerziell erhältliche Cumalinsäure (Aldrich, ACROS, etc.) beispielsweise mit Orthoameisensäuretrimethylester in Gegenwart vom Methanol und konz. Schwefelsäure zum auch kommerziell erhältlichen (2*E*,4*Z*)-4-(Methoxymethylene)-2-pentenedionsäre-1,5-dimethylester (MPDE, Chemieliva, (CAS Registry Number 114106-77-3: (2E,4Z)-4-(Methoxymethylene)-2-pentenedisäure-1,5-dimethylester) umgesetzt, der in einem zweiten Reaktionsschritt mit heterocyclischen Aminen (R-NH₂) zu Verbindungen der Formel (II) reagiert (Schema 4).

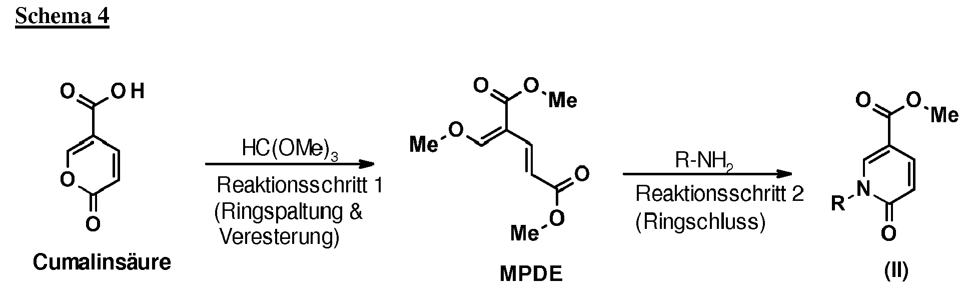

Im Reaktionsschema 4 hat R die oben genannte Bedeutung.

Wird beim Verfahren nach Methode A als Verbindung der Formel (II) 6-Oxo-[6'-trifluormethyl-1(2H),3'-bipyridine]-3-carbonsäuremethylester (R = 6-Trifluormethyl-pyridin-3-yl) (II-1) eingesetzt, so entsteht durch Umsetzung mit einem Alkalimetallhydroxid, beispielsweise Lithiumhydroxid, zunächst 6-Oxo-[6'-trifluormethyl-1(2*H*),3'-bipyridine]-3-carbonsäure (III-1) (CAS Registry Number 1556335-85-3: erhältlich bei Aurora Building Blocks 2), die im zweiten Reaktionsschritt, beispielsweise mit 6-(Trifluoromethyl)-3-pyridinamin, zu dem [6'-Trifluormethyl-[1(2*H*), 3'-bipyridin]-2-on (I-1), reagiert. (Schema 5, vgl. Herstellungsbeispiel 1).

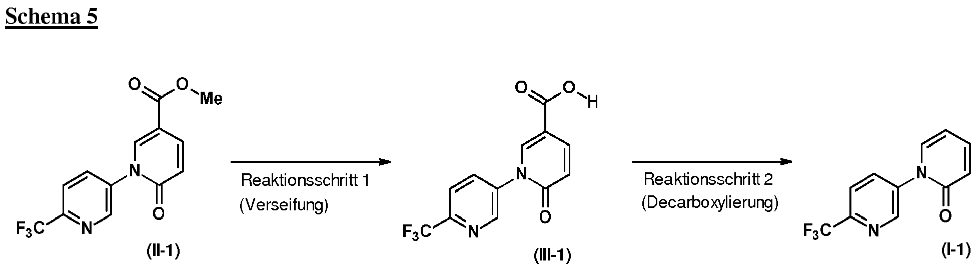

### Reaktionsschritt 1

Reaktionsschritt 1 (Verseifung oder Esterspaltung) wird in Gegenwart einer Base durchgeführt. Geeignet sind anorganische Basen wie Lithiumhydoxid, Natriumhydoxid, Kaliumhydroxid und Caesiumhydroxid. Bevorzugt werden Alkalimetallhydroxide, wie beispielsweise Lithiumhydroxid, eingesetzt. Reaktionsschritt 1 wird in der Regel unter Normaldruck durchgeführt. Das molare Verhältnis des Metallhydroxids zur Verbindung der Formel (II) liegt im Bereich von etwa 10 bis etwa 1.

Vorzugsweise liegt es im Bereich von etwa 8 bis etwa 2, besonders bevorzugt im Bereich von etwa 5 bis 3.

Der Einsatz größerer Mengen an Alkalimetallhydroxiden ist grundsätzlich möglich, jedoch in der Regel unwirtschaftlich.

Geeignete Lösungs- bzw. Verdünnungsmittel für den Reaktionsschritt 1 sind Alkohole und Wasser bzw. Gemische von Alkoholen und Wasser.

Der Reaktionsschritt 1 wird gewöhnlich in einem Temperaturbereich von 20 °C bis 120 °C und gegebenenfalls in Gegenwart eines Lösungs- oder Verdünnungsmittel durchgeführt. Vorzugsweise wird die Umsetzung bei etwa 75 °C in einem Gemisch aus Methanol und Wasser durchgeführt. Vorzugsweise verwendet man ein Gemisch, das aus 2 Teilen Alkohol und 1 Teil Wasser besteht, man kann auch ein Gemisch einsetzen, das aus 3 Teilen Alkohol und 1 Teil Wasser besteht.

### Reaktionsschritt 2

Im zweiten Reaktionsschritt werden die Verbindungen der Formel (III) mit einem Metallpulver, Metalloxid oder Metallcarbonat in einem hoch siedenden Lösungs- oder Verdünnungsmittel zu Verbindungen der Formel (I) umgesetzt.

Im Reaktionsschritt 2 liegt das molare Verhältnis der Verbindungen der Formel (III) zu Metallpulver, Metalloxid oder Metallcarbonat im Bereich von etwa 6 bis etwa 1. Vorzugsweise liegt es im Bereich von etwa 4,5 bis etwa 1,5, besonders bevorzugt im Bereich von etwa 3,5 bis 2. Der Einsatz größerer Mengen an Metallpulver, Metalloxid oder Metallcarbonat ist grundsätzlich möglich, jedoch in der Regel unwirtschaftlich.

Geeignete Metallpulver, Metalloxide und Metallcarbonate sind Cs₂CO₃, Ag₂CO₃ (vgl. auch Dnyaneshwar N. Garad, J. Org. Chem. 2019, 84, 1863-1870), Cu, Cu₂O, CuCl, CuI, CuO und CuCO₃, bevorzugt werden Cu und Cu₂O eingesetzt.

Die Dauer der Reaktion für den Reaktionsschritt 2 liegt im Bereich von etwa 2 bis etwa 25 Stunden, vorzugsweise im Bereich von 4 bis 20 Stunden.

Der Reaktionsschritt 2 wird gewöhnlich in einem Temperaturbereich von 20 °C bis 220 °C durchgeführt. Vorzugsweise wird die Umsetzung bei 120 °C bis 200 °C in einem hochsiedenden Lösungs- oder Verdünnungsmittel durchgeführt.

Als Lösungs- oder Verdünnungsmittel für den Reaktionsschritt 2 kommen alle unter den Reaktionsbedingungen inerten organischen Lösungsmittel in Betracht, beispielsweise Pyridin, Alkylpyridine, β-Picoline, 2-Methyl-5-Ethyl-Pyridin, Chinolin, Amide (wie N,N-Dimethylformamid, N,N-Dimethylacetamid, N-Methyl-formanilid, N-Methylpyrrolidon, Hexamethylphosphorsäure-triamid), N-methyl, N-Butylimidazol, 1,10-Phenanthroline, N-Methylmorpholin, Wasser oder Gemische der genannten Lösungsmittel oder Gemische der genannten Lösungsmittel mit Wasser.

Bevorzugte Lösungs- bzw. Verdünnungsmittel für den Reaktionsschritt 2 sind Chinolin, N,N-Dimethylacetamid, 1,10-Phenanthroline und Wasser.

Die Dauer der Reaktion für den Reaktionsschritt 2 liegt im Bereich von etwa 1 bis etwa 25 Stunden. Eine längere Reaktionsdauer ist möglich, jedoch wirtschaftlich in der Regel nicht sinnvoll.

Zur Aufarbeitung des Reaktionsgemisches wird das Lösungs- oder Verdünnungsmittel im Vakuum entfernt und der Rückstand beispielsweise mit Essigsäureethylester und IN Salzsäure (1:1) verrührt. Die Verbindungen der Formel (I) können anschließend isoliert und gereinigt werden, beispielsweise durch Abtrennen der ausgefallenen Kristalle (vgl. Herstellungsbeispiel 1) oder durch chromatographische Reinigung.

Die Verbindungen der Formel (I) werden mit guten Ausbeuten und in hoher Reinheit erhalten. Es können signifikant bessere Ausbeuten erzielt werden als mit dem in WO 2017/005673 A1 beschriebenen Verfahren.

### Methode B

Nach Methode B (Schema 6) werden Verbindungen der Formel (II) mit Metallsalzen in einem Lösungs- oder Verdünnungsmittel direkt zu Verbindungen der Formel (I) umgesetzt (Krapcho Reaktion, vgl. J. Org. Chem. 1978, 43, 1, 138-147, https://doi.org/10.1021/jo00395a032).

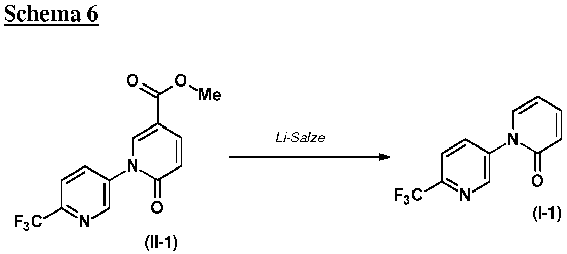

Bevorzugte Metallsalze sind Alkylimetallsalze, beispielsweise LiCl, LiBr und LiI, ganz besonders bevozugt wird LiCl oder LiBr eingesetzt.

Das molare Verhältnis der Verbindungen der Formel (III) zu dem Alkalimetallsalzen liegt im Bereich von etwa 20 bis 0,5.

Bevorzugte Lösungs- bzw. Verdünnungsmittel für das erfindungsgemäßen Verfahren (Methode B) sind DMSO/Wasser Gemische.

Das erfindungsgemäße Verfahren (Methode B) wird gewöhnlich in einem Temperaturbereich von 120 °C bis 200 °C durchgeführt. Vorzugsweise wird die Reaktion bei 120 °C bis 140 °C in einem hochsiedenden

Lösungs- oder Verdünnungsmittel durchgeführt. Die Reaktion kann im offenen System oder unter Druck (Autoklav) durchgeführt werden.

### Erläuterung der Verfahren und Zwischenprodukte

Die Produkte wurden mittels ¹H-NMR Spektroskopie und/oder LC/MS (Liquid Chromatography Mass Spectrometry) charakterisiert.

Die Bestimmung der logP Werte erfolgte gemäß OECD Guideline 117 (EC Directive 92/69/EEC) durch HPLC (High Performance Liquid Chromatography) an reversed-phase (RP) Säulen (C18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker Avance 400, ausgestattet mit einem Durchflussprobenkopf (60 µl Volumen), bestimmt. In Einzelfällen wurden die NMR Spektren mit einem Bruker Avance II 600 gemessen.

### Beispiele:

Die vorliegende Erfindung wird anhand der nachfolgenden Beispiele näher erläutert, ohne die Erfindung dabei auf diese einzuschränken.

### Beispiel 1: (Methode A)

### [6'-Trifluormethyl-[1(2H), 3'-bipyridin]-2-on (I-1)

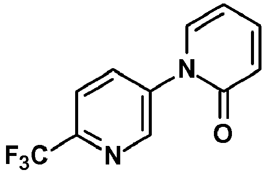

### Schritt 1: Synthese von Verbindungen der Formel (III)

### 6-Oxo-[6'-trifluormethyl-1(2H),3'-bipyridine] -3-carbonsäure (III-1)

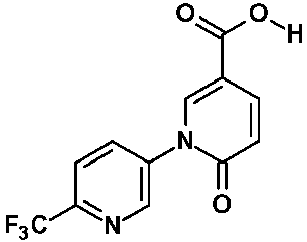

5,0 g (16,7 mmol) 6-Oxo-[6'-trifluormethyl-1(2*H*),3'-bipyridine]-3-carbonsäuremethylester (II-1) wurden in einem Gemisch aus 150 ml Methanol und 50 ml Wasser verrührt und mit 1,20 g (50,3 mmol) Lithiumhydroxid versetzt. Anschließend wurde der Reaktionsansatz 5 Stunden bei 75 °C gerührt. Danach wurde das Reaktionsgemisch mit IN wässriger Salzsäure angesäuert und das Methanol im Vakuum entfernt. Die dabei ausgefallenen Kristalle wurden abgetrennt, in Aceton gelöst und die Lösung wurde erneut im Vakuum eingeengt. Man erhielt 3,43 g (71,6 % Ausbeute) 6-Oxo-[6'-trifluormethyl-1(2*H*),3'-bipyridine]-3-carbonsäure (III-1).

| | |
|---|---|
| Summenformel: C₁₂H₇F₃N₂O₃ | Molgewicht: 284,19 g/mol |
| HPLC-MS (ESI Positiv): 285,1 (M+1); Rₜ = 0,63; 0,65 min | LogP-Wert = 1.20, 1.22 |

### Schritt 2: Synthese von Verbindungen der Formel (I)

### [6'-Trifluormethyl-[1(2H), 3'-bipyridin]-2-on (I-1)

500 mg (1,76 mmol) 6-Oxo-[6'-trifluormethyl-1(2*H*),3'-bipyridine]-3-carbonsäure (III-1) wurden in 2 ml Chinolin verrührt, mit 50,3 mg (0,79 mmol) Kupferpulver versetzt und 18 Stunden bei 195 °C gerührt. Nach dem Abkühlen wurde der Reaktionsansatz in einem Gemisch aus Essigsäureethylester und IN Salzsäure (1:1) verrührt. Danach wurde die organische Phase abgetrennt, über Natriumsulfat getrocknet und im Vakuum eingeengt. Anschließend wurde das zurückbleibende Rohprodukt mittels Säulenchromatographie (MPLC) an Kieselgel (Laufmittel: Cyclohexan - Aceton Gradient) gereinigt. Man erhielt 400 mg (100 % Reinheit, 94,6 % Ausbeute) [6'-Trifluormethyl-[1(2*H*), 3'-bipyridin]-2-on (I-1)

| | |
|---|---|
| Summenformel: C₁₁H₇F₃N₂O | Molgewicht: 240,18 g/mol |
| HPLC-MS (ESI Positiv): 241,1 (M+1); Rₜ = 0,67; 0,68 min | LogP-Wert = 1.29, 1.31 |

¹H-NMR (400 MHz, DMSO-d₆), δ[ppm] = 8,8988 (12,41), 8,8936 (12,38), 8,2739 (6,04), 8,2680 (5,91), 8,2530 (7,80), 8,2471 (7,68), 8,1176 (16,00), 8,0966 (12,31), 7,8170 (7,86), 7,8119 (8,35), 7,7997 (8,27), 7,7947 (8,30), 7,6019 (4,46), 7,5977 (4,42), 7,5854 (4,95), 7,5799 (8,22), 7,5744 (4,72), 7,5621 (4,92), 7,5579 (4,54), 6,5696 (10,76), 6,5463 (10,25), 6,4338 (5,43), 6,4323 (5,53), 6,4170 (10,26), 6,4155 (10,24), 6,4001 (5,20), 6,3985 (5,21), 3,3303 (31,72), 2,6722 (0,44), 2,5248 (1,62), 2,5074 (55,48), 2,5032 (70,64), 2,4989 (52,20), 2,3301 (0,42), 2,0863 (0,33), 1,3975 (1,74), 0,0076 (2,66), -0,0002 (54,73), -0,0074 (2,12).

### Beispiel 2: (Vergleichsbeispiel, beschrieben in WO 2017/005673 A1)

### [6'-Trifluormethyl-[1(2H), 3'-bipyridin]-2-on (1)

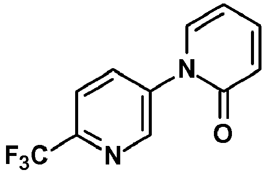

Zu einer Lösung von 1,0 g (4.42 mmol) 5-Brom-2-trifluormethyl-pyridin in 6 ml *N,N*-Dimethyl-formamid wurden unter Argonatmosphäre 378,7 mg (3,98 mmol) 2(1*H*)-Pyridinon, 550,3 mg (3,98 mmol) Kaliumcarbonat und 42,1 mg (0,22 mmol) Kupfer(I)-iodid gegeben. Danach wurde das Reaktionsgemisch 20 Stunden bei 120 °C gerührt. Zur Aufarbeitung wurde der Reaktionsansatz nach dem Abkühlen in 60 ml Wasser verrührt und dreimal mit Essissäureethylester extrahiert. Die organische Phase wurde abgetrennt, getrocknet und im Vakuum eingeengt. Der verbleibende Rückstand wurde mittels Säulenchromatographie an Kieselgel gereinigt. Man erhielt 420 mg (100 % Reinheit, 35,6 % Ausbeute) 6'-Trifluormethyl-[1(2*H*),3'-bipyridin]-2-on.

### Synthese der Ausgangsverbindungen

### 6-Oxo-[6'-trifluormethyl-1(2H),3'-bipyridin]-3-earbonsäure methylester (II-1)

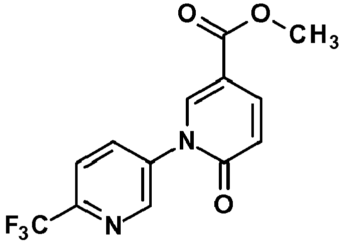

### Schritt 1: Synthese von (2E,4Z)-4-(Methoxymethylen)-2-pentendionsäure-1,5-dimethylester

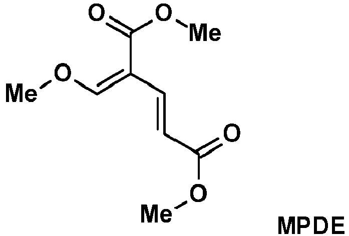

Zu einer Lösung von 25 g (178,4 mmol) Cumalinsäure und 450 ml Orthoameisensäuretrimethylester in 150 ml Methanol wurden bei Raumtemperatur 28,5 ml konz. Schwefelsäure getropft. Danach wurde 48 Stunden bei Rückflußtemperatur gerührt. Nach dem Abkühlen wurde mit 500 ml Essigsäureethylester versetzt und vorsichtig mit wässriger Natriumhydrogencarbonatlösung extrahiert. Zur Aufarbeitung wurde die organische Phase abgetrennt, über Natriumsulfat getrocknet und mit Kieselgur im Vakuum eingeengt. Die Reinigung erfolgte mittels Säulenchromatographie (MPLC) an Kieselgel (Laufmittel Gradient: Cyclohexan - Aceton Gradient). Man erhielt 25 g (70,1 % der Theorie) (2*E*,4*Z*)-4-(Methoxymethylen)- 2-pentendionsäure-1,5 -dimethylester.

| | |
|---|---|
| Summenformel: C₉H₁₂O₅ | Molgewicht: 200,19 g/mol |
| HPLC-MS (ESI Positiv): 201,2 (M+1); Rₜ = 0,85; 0,86 min | LogP-Wert = 1.73, 1.75 |

### 6-Oxo-[6'-trifluormethyl-1(2H),3'-bipyridine]-3-carbonsäure methylester (II-1)

14,8 g (74,0 mmol) (2*E*,4*Z*)-4-(Methoxymethylen)-2-pentendionsäure-1,5-dimethylester (MPDE) wurden mit 12,0 g (74,0 mmol) 5-Amino-2-trifluormethyl-pyridin in 146,5 ml *N,N*-Dimethylformamid 18 Stunden bei 150 °C gerührt. Nach dem Abkühlen wurden die ausgefallenen Kristalle abgetrennt und getrocknet.

| | |
|---|---|
| Summenformel: C₁₃H₉F₃N₂O₃ | Molgewicht: 298,22 g/mol |
| HPLC-MS (ESI Positiv): 299,1 (M+1); Rₜ = 0,87; 0,88 min | LogP-Wert = 1.77, 1.80 |

## Patentansprüche

1. Verfahren zur Herstellung von Verbindungen der Formel (I) 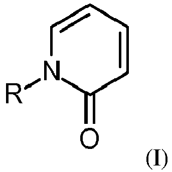 worin
R für einen Rest B aus der Reihe 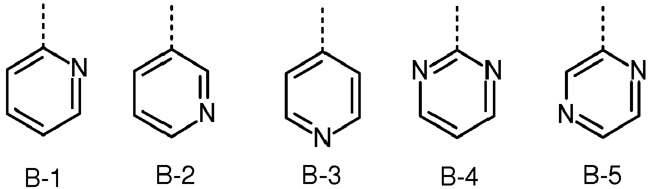 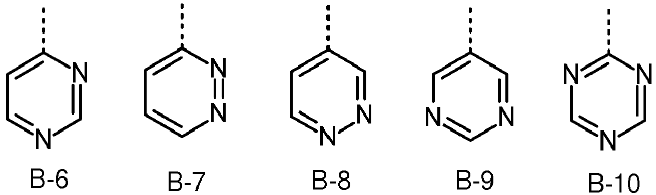 steht, wobei diese Reste n Substituenten Y tragen und die gestrichelte Linie die Bindung zum Stickstoffatom im Rest A bedeutet,
Y für einen Rest aus der Reihe Halogen, Cyano, Nitro, Amino, Alkyl, Alkenyl, Alkinyl, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl, Alkoxycarbonyl, Alkylcarbonyl, Cycloalkylcarbonyl, Alkylamino, Dialkylamino, Alkylaminosulfonyl, Dialkylaminosulfonyl, Alkylaminocarbonyl, Dialkylaminocarbonyl, Alkylcarbonylamino, Alkoxyalkylcarbonylamino, Halogenalkylcarbonylamino und jeweils gegebenenfalls substituiertes Aryl und Hetaryl steht und
n für eine Zahl aus der Reihe 0, 1 und 2 steht,
**dadurch gekennzeichnet, dass** in einem ersten Schritt Verbindungen der Formel (II) 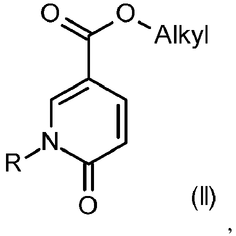 worin
Alkyl für C₁-C₄-Alkyl steht und
R die oben genannten Bedeutungen hat,
mit einem Alkalimetallhydroxid in Gegenwart eines Verdünnungsmittels in Verbindungen der Formel (III) 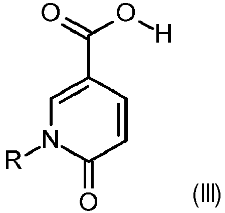 worin
R die oben genannten Bedeutungen hat,
überführt werden und diese anschließend in einem zweiten Reaktionsschritt in Gegenwart eines Metallpulvers, Metalloxids oder Metallcarbonats und einem hoch siedenden Lösungs- oder Verdünnungsmittel zu Verbindungen der Formel (I) umgesetzt werden (Methode A) oder,
dass Verbindungen der Formel (II) 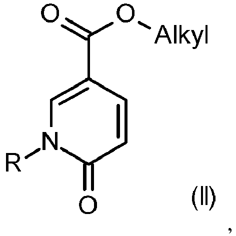 worin
Alkyl für C₁-C₄-Alkyl steht und
R die oben genannten Bedeutungen hat,
mit Metallsalzen in einem Lösungs- oder Verdünnungsmittel zu Verbindungen der Formel (I) umgesetzt werden (Methode B).

2. Verfahren nach Anspruch 1, Methode A.

3. Verfahren nach Anspruch 1, Methode B.
